# EUROPEAN PATENT APPLICATION

(11) **EP 4 663 629 A1**
(43) Date of publication of application: **17.12.2025**
(21) Application number: 24753416.7
(22) Date of filing: 08.02.2024
(51) Int. Cl.: C07D 231/20, C07D 231/22, C07D 401/12, C07D 487/04

(54) **FLUORINATED PYRAZOLONE COMPOUND AND METHOD FOR PRODUCING SAME**

(30) Priority: 09.02.2023 JP 2023018687
(71) Applicant: Unimatec Co., Ltd., Tokyo 105-0012 (JP)
(72) Inventor: SEINO, Junya, Kitaibaraki-shi, Ibaraki 319-1593 (JP); AOTSU, Rie, Kitaibaraki-shi, Ibaraki 319-1593 (JP); TAKAHASHI, Yusuke, Kitaibaraki-shi, Ibaraki 319-1593 (JP)
(74) Representative: Global IP Europe Patentanwaltskanzlei
(86) International application number: PCT/JP2024/004259
(87) International publication number: WO 2024/166973

(57) **Abstract**

To provide a novel fluorine-containing 1,2-disubstituted-3-pyrazolone compound having a trifluoromethyl group at the 4-position and a specific substituent at the 5-position and a production method capable of simply producing the fluorine-containing pyrazolone compound without the formation of an O-substituted product as a by-product.

A fluorine-containing pyrazolone compound represented by the following formula (1): 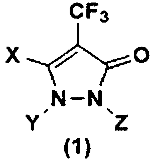 wherein X represents a halogen atom, -OA¹, -S(O₁)A¹, where l is an integer of 0 to 2, -O-NA¹A², -NA¹A², -NA³-NA¹A², or -N=A⁴; Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring; and A¹, A², A³, and A⁴ each independently represent an organic group having 1 to 12 carbon atoms.

## Description

### Technical Field

The present invention relates to a fluorine-containing pyrazolone compound and a method for producing the same.

### Background Art

Compounds having a pyrazolone ring have been known to exhibit various pharmacological actions, among which 3-pyrazolone compounds having substituents at the 1- and 2-positions have been used in the pharmaceutical and agrochemical fields. Examples of compounds having a 1,2-disubstituted-3-pyrazolone ring include, for pharmaceuticals, anti-inflammatory agents such as Nifenazone, Propyphenazone, Dipyrone, Antipyrine, and Aminopyrine. Moreover, examples of agrochemicals include a fungicide such as Fenpyrazamine and a herbicide such as Pinoxaden.

In this context, there has been growing interest in the introduction of substituents into the 4- and 5-positions of the 1,2-disubstituted-3-pyrazolone ring, and particularly in the development of methods for synthesizing a 1,2-disubstituted-3-pyrazolone compound having a trifluoromethyl group at the 4-position and a specific substituent at the 5-position, such as a heteroatom-containing substituent.

Patent Literature 1 (Japanese Patent Laid-Open No. 61-118371) discloses a method for preparing a 3-pyrazolone compound having a substituent at the 1-position, a trifluoromethyl group at the 4-position, and a heteroatom substituent at the 5-position. Non Patent Literatures 1 and 2 disclose the introduction of a substituent onto a pyrazolone ring of a compound having the pyrazolone ring.

### Document List

### Patent Literature

Patent Literature 1: Japanese Patent Application Publication No. 61-118371

### Non Patent Literatures

Non Patent Literature 1: Bioorganic & Medicinal Chemistry, 2017, volume 25, pages 5327-5340
Non Patent Literature 2: Journal of Molecular Structure, 2020, volume 1215, page 128272

### Summary of Invention

### Technical Problem

It is considered that a desired 1,2-disubstituted-3-pyrazolone compound is produced by introducing a substituent into the 2-position of the pyrazolone ring of the compound obtained by the production method disclosed in Patent Literature 1. However, Non Patent Literature 1 reports that, in the reaction of introducing a substituent into the 2-position of the pyrazolone ring of a compound having a 3-pyrazolone ring structure substituted at the 1-position, there may be competition with the reaction of introducing a substituent onto the oxygen at the 3-position of the pyrazolone ring. Similarly, Non Patent Literature 2 reports that, in the reaction of introducing a substituent into the 1-position of the pyrazolone ring of a compound having a 3-pyrazolone ring structure substituted at the 2-position, there may be competition with the reaction of introducing a substituent onto the oxygen at the 3-position of the pyrazolone ring. According to the disclosures of these documents, therefore, when an attempt is made to obtain a 1,2-disubstituted-3-pyrazolone compound from a 3-pyrazolone compound substituted at the 1- or 2-position, the formation of a compound in which a substituent is introduced onto the oxygen at the 3-position (O-substituted product) as a by-product cannot be suppressed, and there has been room for investigation in terms of efficiently producing the desired 1,2-disubstituted-3-pyrazolone compound.

Thus, the present inventors have discovered that reacting certain raw materials enables a 1,2-disubstituted-3-pyrazolone compound having a trifluoromethyl group at the 4-position and a specific substituent, such as a heteroatom-containing substituent at the 5-position, to be obtained without producing an O-substituted product as a by-product, thereby completing the present invention. In other words, it is an object of the present invention to provide a 1,2-disubstituted-3-pyrazolone compound having a trifluoromethyl group at the 4-position and a specific substituent at the 5-position, which has not been known before, and a production method capable of simply producing the pyrazolone compound without the formation of an O-substituted product as a by-product.

### Solution to Problem

The gist configuration of the present invention is as follows:
[1] A fluorine-containing pyrazolone compound represented by the following formula (1): 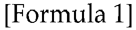 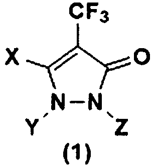 wherein
   X represents a halogen atom, -OA¹, -S(Oₗ)A¹, where l is an integer of 0 to 2, -O-NA¹A², -NA¹A², -NA³-NA¹A², or -N=A⁴,
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring, and
   A¹, A², A³, and A⁴ each independently represent an organic group having 1 to 12 carbon atoms.
[2] A method for producing a fluorine-containing pyrazolone compound, comprising a step of reacting a fluoroisobutanoic acid fluoride derivative represented by the following formula (2) with a compound represented by the following formula (6) or a salt thereof to obtain a fluorine-containing pyrazolone compound represented by the following formula (1a): 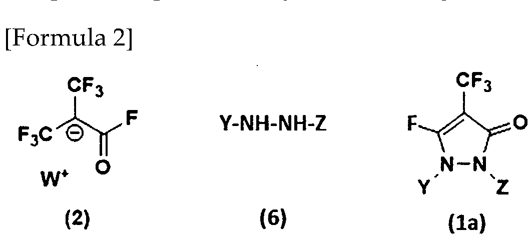 wherein
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   A¹ represents an organic group having 1 to 12 carbon atoms, and
   W⁺ represents an ammonium cation, an imidazolium cation, a pyridinium cation, or a phosphonium cation.
[3] A method for producing a fluorine-containing pyrazolone compound, comprising a step of reacting a fluoroisobutanoic acid fluoride derivative represented by the following formula (2) with a compound represented by the following formula (5) and a compound represented by the following formula (6) or a salt thereof to obtain a fluorine-containing pyrazolone compound represented by the following formula (1b): 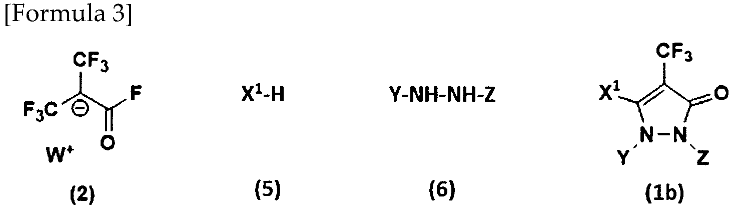 wherein
   X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   A¹, A², A³, and A⁴ each independently represent an organic group having 1 to 12 carbon atoms, and
   W⁺ represents an ammonium cation, an imidazolium cation, a pyridinium cation, or a phosphonium cation.
[4] A method for producing a fluorine-containing pyrazolone compound, comprising a step of reacting a fluoroisobutene derivative represented by the following formula (3) with a compound represented by the following formula (6) or a salt thereof to obtain a fluorine-containing pyrazolone compound represented by the following formula (1a): 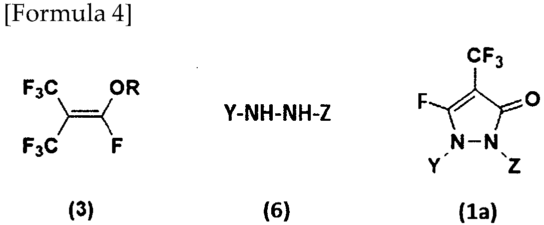 wherein
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   A¹ represents an organic group having 1 to 12 carbon atoms, and
   R represents a hydrocarbon group having 1 to 12 carbon atoms.
[5] A method for producing a fluorine-containing pyrazolone compound, comprising a step of reacting a fluoroisobutene derivative represented by the following formula (3) with a compound represented by the following formula (5) and a compound represented by the following formula (6) or a salt thereof to obtain a fluorine-containing pyrazolone compound represented by the following formula (1b): 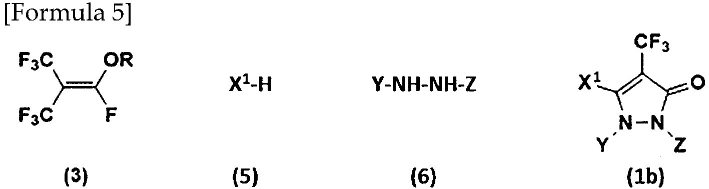 wherein
   X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   A¹, A², A³, and A⁴ each independently represent an organic group having 1 to 12 carbon atoms, and
   R represents a hydrocarbon group having 1 to 12 carbon atoms.
[6] A method for producing a fluorine-containing pyrazolone compound, comprising a step of reacting a fluoroisobutane derivative represented by the following formula (4) with a compound represented by the following formula (6) or a salt thereof to obtain a fluorine-containing pyrazolone compound represented by the following formula (1a): 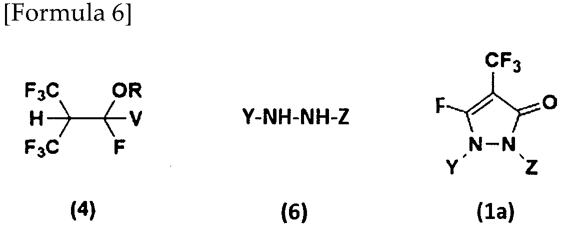 wherein
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   R represents a hydrocarbon group having 1 to 12 carbon atoms,
   V represents a halogen atom, -OA⁵, or -SOₘA⁵, where m is an integer of 0 to 2, and
   A¹ and A⁵ each independently represent an organic group having 1 to 12 carbon atoms.
[7] A method for producing a fluorine-containing pyrazolone compound, comprising a step of reacting a fluoroisobutane derivative represented by the following formula (4) with a compound represented by the following formula (5) and a compound represented by the following formula (6) or a salt thereof to obtain a fluorine-containing pyrazolone compound represented by the following formula (1b): 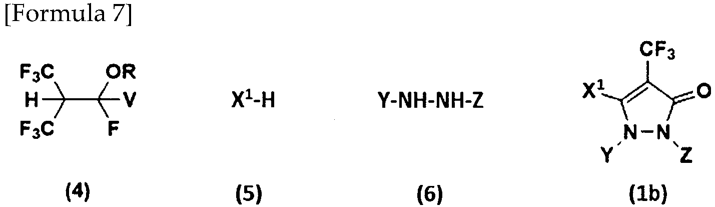 wherein
   X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   R represents a hydrocarbon group having 1 to 12 carbon atoms,
   V represents a halogen atom, -OA⁵, or -SOₘA⁵, where m is an integer of 0 to 2, and
   A¹, A², A³, A⁴, and A⁵ each independently represent an organic group having 1 to 12 carbon atoms.

### Effects of Invention

It is possible to provide a novel fluorine-containing 1,2-disubstituted-3-pyrazolone compound having a trifluoromethyl group at the 4-position and a specific substituent at the 5-position and a production method capable of simply producing the fluorine-containing pyrazolone compound without the formation of an O-substituted product as a by-product.

### Description of Embodiments

### (Fluorine-containing Pyrazolone Compound)

The fluorine-containing pyrazolone compound of the present invention is represented by the following formula (1). 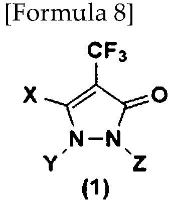 wherein
X represents a halogen atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring, and
A¹, A², A³, and A⁴ each independently represent an organic group having 1 to 12 carbon atoms.

Since the fluorine-containing pyrazolone compound of the present invention has specific substituents (-CF₃ and -X) at the 4- and 5-positions of the pyrazolone ring, the fluorine-containing pyrazolone compound can exhibit an excellent effect from the viewpoint of structural expandability. In particular, desired biological activities (e.g., inhibitory activity on a hormone or enzyme, fungicidal activity, insecticidal activity, or herbicidal activity) can be expected. In particular, examples of the fungicidal activity include fungicidal activity on bacteria that have a harmful effect on the human body and crops such as rice. In addition, since the substituents at the 4- and 5-positions of the pyrazolone ring are different groups (-CF₃ and -X) from each other, these groups can be readily derivatized into asymmetric structures through elimination or reaction and can be expected to be used as intermediates. More specifically, -CF₃ can be substituted by reacting the fluorine-containing pyrazolone compound to obtain a derivative. Moreover, -X can be modified by reacting the fluorine-containing pyrazolone compound under basic conditions to obtain a derivative. A fluorine-containing pyrazolone compound of one embodiment is useful in fields of electronic materials, such as organic semiconductors and liquid crystals.

The group X represents a halogen atom, -OA¹, -S(Oₜ)A¹, where l is an integer of 0 to 2, -O-NA¹A², -NA¹A², -NA³-NA¹A², or -N=A⁴. Although A¹, A², A³, and A⁴ each independently represent an organic group having 1 to 12 carbon atoms, A¹, A², and A³ are monovalent groups, while A⁴ is a divalent group. A¹, A², A³, and A⁴ may be organic groups having 1 to 12 carbon atoms and may contain, in addition to carbon atoms, a hydrogen atom, an oxygen atom, a sulfur atom, or a nitrogen atom. Examples of A¹, A², and A³ include chain hydrocarbon groups having 1 to 12 carbon atoms (e.g., an alkyl group, an alkenyl group, and an alkynyl group), aromatic hydrocarbon groups (e.g., a phenyl group), alicyclic hydrocarbon groups (e.g., a cyclopropyl group, a cyclobutyl group, a cyclohexyl group, a cyclopentyl group, and an adamantyl group), heterocyclic groups, - COA⁶, -CₙH₂ₙ-A⁷, -CₙH₂ₙ-O-A⁷ (where n is an integer of 1 or more and 11 or less, A⁶ is an organic group having 1 to 11 carbon atoms, and A⁷ is an organic group having 1 to 12-n carbon atoms; examples of A⁶ include a furyl group, a benzofuryl group, and a thienyl group; and examples of A⁷ include an alkyl group, phenyl group, pyridyl group, naphthyl group, and thienyl group having 1 to 12-n carbon atoms). The chain hydrocarbon group may be a branched chain hydrocarbon group or an unbranched chain hydrocarbon group. The aromatic hydrocarbon group may be an aromatic hydrocarbon group having a substituent or an aromatic hydrocarbon group having no substituent. The aromatic hydrocarbon group may also have a condensed polycyclic structure. The alicyclic hydrocarbon group may be an alicyclic hydrocarbon group having a substituent or an alicyclic hydrocarbon group having no substituent. The alicyclic hydrocarbon group may also have a bridged ring structure. The heterocyclic group may be any heterocyclic group containing one or two or more heteroatoms (an oxygen atom, a sulfur atom, and a nitrogen atom) as ring atoms, and may have a monocyclic structure or a fused polycyclic structure. Examples of A⁴ include a cyclopentylidene group, a cyclohexylidene group, a diphenylmethylene group, a dibutylmethylene group, =CN(A⁸)₂N(A⁹)₂ (where A⁸ and A⁹ are each independently an alkyl group having 1 to 8 carbon atoms), a di(ethoxy)methylene group, and a di(ethylthio)methylene group. The group X is preferably a halogen atom, -O-NA¹A², or -NA¹A². Examples of the halogen atom as the group X include a fluorine atom (F), a chlorine atom (Cl), a bromine atom (Br), and an iodine atom (I), and a fluorine atom is preferable. Moreover, A¹, A², and A³ are each preferably an alkyl group, -COA⁶, -CₙH₂ₙ-A⁷, or -CₙH₂ₙ-O-A⁷.

Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring. Y and Z are each independently preferably a phenyl group, an alkyl group having 1 to 12 carbon atoms, -COA¹ (A¹ is an alkyl group having 1 to 12 carbon atoms), or - COOA¹ (A¹ is an alkyl group having 1 to 12 carbon atoms), or a benzyl group, or Y and Z are preferably bonded to each other to form a ring. When Y and Z are bonded to each other to form a ring, examples of the ring include a ring in which Y and Z together constitute -CₙH₂ₙ- to form a ring structure between the nitrogen atoms at the 1- and 2-positions of the pyrazolone ring. In this case, a ring having a structure of -Y-Z-CₙH₂ₙ- is formed. Although the number of ring atoms forming such a ring is not limited, the number of ring atoms is preferably 4 to 12, more preferably 5 to 10, and still more preferably 6 to 8. In addition, the ring formed by the bonding of Y and Z may or may not have a substituent. When the ring formed by the bonding of Y and Z has a substituent, examples of the substituent include =O, an alkyl group having 1 to 12 carbon atoms, and a halogen atom.

### (Method for Producing Fluorine-containing Pyrazolone Compound)

A method for producing a fluorine-containing pyrazolone compound of one embodiment includes:
(a) a step of reacting a fluoroisobutanoic acid fluoride derivative represented by the following formula (2) with a compound represented by the following formula (6) or a salt thereof to obtain a fluorine-containing pyrazolone compound represented by the following formula (1a): 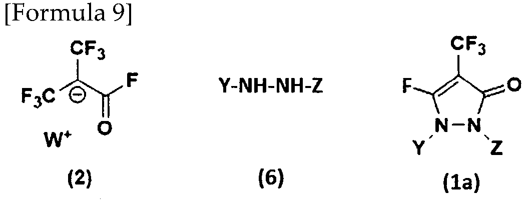 wherein
   Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
   A¹ represents an organic group having 1 to 12 carbon atoms,
   W⁺ represents an ammonium cation, an imidazolium cation, a pyridinium cation, or a phosphonium cation.
   A¹ may be the same group as A¹ above.
   Examples of the ammonium cation include a triethylmethylammonium cation, a butyltrimethylammonium cation, and an N-methylquinuclidinium cation.
   Examples of the imidazolium cation include a 1-butyl-3-methylimidazolium cation, a 1,3-dimethylimidazolium cation, and a 1-benzyl-3-methylimidazolium cation.
   Examples of the pyridinium cation include a 1-methyl-4-dimethylaminopyridinium cation, a 1-methylpyridinium cation, and a 1,4-dimethylpyridinium cation.
   Examples of the phosphonium cation include a tricyclohexylmethylphosphonium cation, a triphenylmethylphosphonium cation, and a tributylmethylphosphonium cation.
   Examples of salts of the compound represented by the formula (6) include hydrochloride, trifluoroacetate, and p-toluenesulfonate of the compound represented by the formula (6).

Preferably, the step (a) of obtaining a fluorine-containing pyrazolone compound is preferably carried out in the presence of a fluoride ion scavenger. It is preferable to react the fluoroisobutanoic acid fluoride derivative represented by the formula (2) with the compound represented by the formula (6) or a salt thereof in the presence of a fluoride ion scavenger. The fluoride ion scavenger is not particularly limited as long as it is a substance having a function of capturing fluoride ions, and examples of the fluoride ion scavenger include lithium, sodium, magnesium, potassium, calcium, tetramethylammonium, trifluoroacetic acid, heptafluorobutyric acid, methanesulfonic acid, ethanesulfonic acid, benzenesulfonic acid, toluenesulfonic acid, trifluoromethanesulfonic acid, nonafluorobutanesulfonic acid, bis(trifluoromethanesulfonyl)imide, bis(nonafluorobutanesulfonyl)imide, N,N-hexafluoropropane-1,3-disulfonylimide, tetraphenylboric acid, tetrakis[3, 5-bis(trifluoromethyl)phenyl]boric acid, and tetrakis(pentafluorophenyl)borate. It is considered that the cation derived from the fluoride ion scavenger captures fluoride ions liberated from the fluoroisobutanoic acid fluoride derivative represented by the formula (2) during the reaction and precipitates the fluoride ions as a salt having low solubility in an organic solvent to promote the reaction, enabling the fluorine-containing pyrazolone compound represented by the formula (1a) to be obtained in a high yield.

The reaction in (a) above, in which a fluoroisobutanoic acid fluoride derivative represented by the formula (2) is reacted with a compound represented by the formula (6) to obtain a fluorine-containing pyrazolone compound represented by the formula (1a), is represented by the following reaction formula (A).

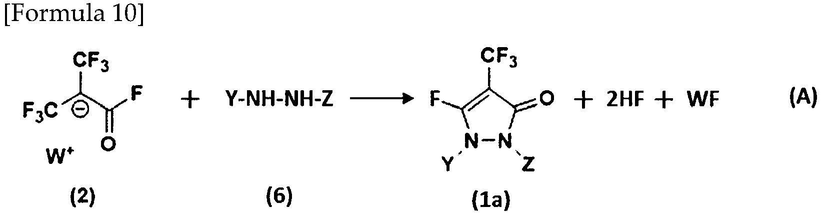

In the method for producing a fluorine-containing pyrazolone compound of one embodiment, the reaction (A) can be carried out in a single step. Consequently, the fluorine-containing pyrazolone compound of the formula (1a) can be obtained in a simplified manner. In the reaction of (a) above, the compound of the formula (1a) is formed from the fluoroisobutanoic acid fluoride derivative represented by the formula (2) and the compound represented by the formula (6). At the 4- and 5-positions of the pyrazolone structure are located - CF₃ and -F, respectively, derived from the fluoroisobutanoic acid fluoride derivative.

The reaction in (a) above is preferably carried out in the presence of a hydrogen halide scavenger. The hydrogen halide scavenger is a substance that has the function of capturing hydrogen fluoride (HF) formed from a hydrogen atom derived from the compound of the formula (6) and a fluorine atom derived from the fluoroisobutanoic acid fluoride derivative of the formula (2) in the reaction formula (A). Examples of the hydrogen halide scavenger that can be used include sodium hydroxide, potassium hydroxide, calcium hydroxide, sodium carbonate, potassium carbonate, sodium fluoride, potassium fluoride, and organic nitrogen derivatives such as pyridine, triethylamine, diisopropylethylamine, diazabicyclononene, diazabicycloundecene, methyltriazabicyclodecene, diazabicyclooctane, and phosphazene base.

A reaction temperature during the reaction in (a) above is preferably - 20°C or higher and lower than the boiling point temperature of an organic solvent, more preferably 0 to 50°C, and still more preferably 10 to 30°C. A reaction time during the reaction in (a) above is preferably 0.5 to 48 hours, more preferably 1 to 36 hours, and still more preferably 10 to 25 hours.

Examples of the solvent to be used in the reaction of (a) above include ethers such as tetrahydrofuran, diethyl ether, dioxane, monoglyme, diglyme, triglyme, and tetraglyme; aromatic hydrocarbons such as benzene, toluene, and xylene; a nitrile such as acetonitrile; and aprotic polar solvents such as dimethylformamide, dimethylacetamide, methylpyrrolidone, dimethylethyleneurea, tetramethylurea, dimethylsulfoxide, and sulfolane.

A method for producing a fluorine-containing pyrazolone compound of another embodiment includes:
(b) a step of reacting a fluoroisobutanoic acid fluoride derivative represented by the following formula (2) with a compound represented by the following formula (5) and a compound represented by the following formula (6) or a salt thereof to obtain a fluorine-containing pyrazolone compound represented by the following formula (1b): 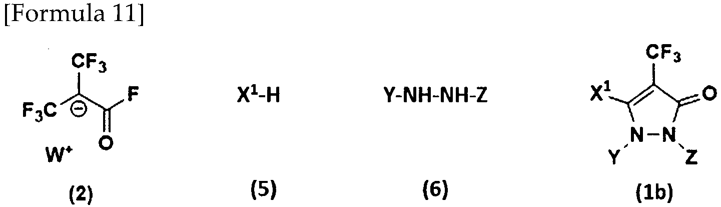
wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹, A², A³, and A⁴ each independently represent an organic group having 1 to 12 carbon atoms, and
W⁺ represents an ammonium cation, an imidazolium cation, a pyridinium cation, or a phosphonium cation.
A¹, A², A³, and A⁴ may be the same groups as A¹, A², A³, and A⁴ above.
Examples of salts of the compound represented by the formula (6) include hydrochloride, trifluoroacetate, and p-toluenesulfonate of the compound represented by the formula (6).

Preferably, the step (b) of obtaining a fluorine-containing pyrazolone compound is preferably carried out in the presence of a fluoride ion scavenger. It is preferable to react the fluoroisobutanoic acid fluoride derivative represented by the formula (2) with the compound represented by the formula (5) and the compound represented by the formula (6) or a salt thereof in the presence of a fluoride ion scavenger. The fluoride ion scavenger is not particularly limited as long as it is a substance having a function of capturing fluoride ions, and examples thereof include the same fluoride ion scavenger to be used in the step (a). It is considered that the cation derived from the fluoride ion scavenger captures fluoride ions liberated from the fluoroisobutanoic acid fluoride derivative represented by the formula (2) during the reaction and precipitates the fluoride ions as a salt having low solubility in an organic solvent to promote the reaction, enabling the fluorine-containing pyrazolone compound represented by the formula (1b) to be obtained in a high yield.

The reaction in (b) above between a fluoroisobutanoic acid fluoride derivative represented by the formula (2) and compounds represented by the formulae (5) and (6) is represented by the following reaction formula (B).

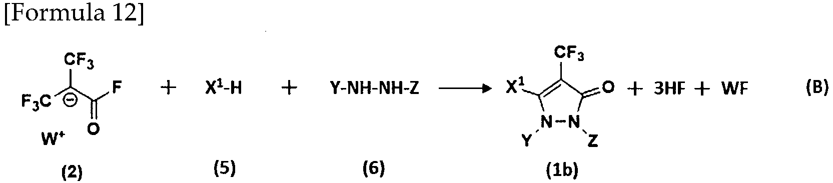

In the method for producing a fluorine-containing pyrazolone compound of another embodiment, the reaction (B) can be carried out in a single step. Consequently, the fluorine-containing pyrazolone compound of the formula (1b) can be obtained in a simplified manner. In the reaction of (b) above, a cyclic pyrazolone ring structure is formed between the fluoroisobutanoic acid fluoride derivative represented by the formula (2) and the compound of the formula (6), and -CF₃ derived from the fluoroisobutanoic acid fluoride derivative and -X¹ derived from the compound of the formula (5) are located at the 4- and 5-positions of the pyrazolone ring structure, respectively.

A reaction temperature during the reaction in (b) above is preferably - 20°C or higher and lower than the boiling point temperature of an organic solvent, more preferably 0 to 50°C, and still more preferably 10 to 30°C. A reaction time during the reaction in (b) above is preferably 0.5 to 48 hours, more preferably 1 to 36 hours, and still more preferably 10 to 25 hours. In the reaction of (b) above, the same hydrogen halide scavenger and solvent as in (a) above can be used.

A method for producing a fluorine-containing pyrazolone compound of another embodiment includes:
(c) a step of reacting a fluoroisobutene derivative represented by the following formula (3) with a compound represented by the following formula (6) or a salt thereof to obtain a fluorine-containing pyrazolone compound represented by the following formula (1a): 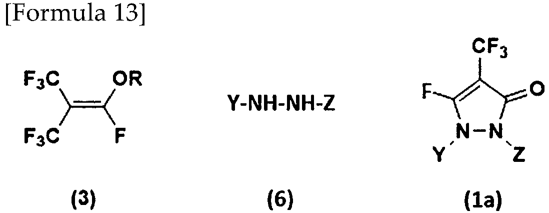
wherein
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹ represents an organic group having 1 to 12 carbon atoms, and
R represents a hydrocarbon group having 1 to 12 carbon atoms.
A¹ may be the same group as A¹ above.
Examples of R include a hydrocarbon group having 1 to 10 carbon atoms among A¹. More specifically, examples of R include a chain hydrocarbon group, aromatic hydrocarbon group, and alicyclic hydrocarbon group having 1 to 10 carbon atoms. Examples of the chain hydrocarbon group include alkyl groups such as a methyl group, an ethyl group, a n-propyl group, an i-propyl group, a n-butyl group, an i-butyl group, a sec-butyl group, a t-butyl group, a pentyl group, a hexyl group, a heptyl group, an octyl group, a nonyl group, and a decyl group; alkenyl groups such as an ethenyl group, a propenyl group, a butenyl group, a pentenyl group, a hexenyl group, a heptenyl group, an octenyl group, a nonenyl group, and a decenyl group; and alkynyl groups such as an ethynyl group, a propynyl group, a butynyl group, a pentynyl group, a hexynyl group, a heptynyl group, an octynyl group, a nonynyl group, and a decynyl group. Examples of the aromatic hydrocarbon group include a phenyl group. Examples of the alicyclic hydrocarbon group include a saturated or unsaturated cyclic hydrocarbon group, and examples of the cyclic hydrocarbon group include a cyclopropyl group, a cyclobutyl group, a cyclohexyl group, a cyclopentyl group, and an adamantyl group. Preferably, R is an alkyl group having 1 to 10 carbon atoms. When R is an alkyl group having 1 to 10 carbon atoms, a raw material of the fluorine-containing pyrazolone compound can be easily prepared.
Examples of salts of the compound represented by the formula (6) include hydrochloride, trifluoroacetate, and p-toluenesulfonate of the compound represented by the formula (6).

The step (c) of obtaining a fluorine-containing pyrazolone compound is preferably carried out in the presence of a nucleophilic reagent. Examples of the nucleophilic reagent include 1-methylimidazole, 4-dimethylaminopyridine, 1,4-diazabicyclo[2.2.2] octane, and triphenylphosphine. The use of the nucleophilic reagent can effectively eliminate the substituent -R from the fluoroisobutene derivative represented by the formula (3) to promote the formation of the fluorine-containing pyrazolone compound represented by the formula (1a). The step (c) of obtaining a fluorine-containing pyrazolone compound is preferably carried out in the presence of a fluoride ion scavenger. It is preferable to react the fluoroisobutene derivative represented by the formula (3) with the compound represented by the formula (6) or a salt thereof in the presence of a fluoride ion scavenger. The fluoride ion scavenger is not particularly limited as long as it is a substance having a function of capturing fluoride ions, and examples thereof include the same fluoride ion scavenger to be used in the step (a). It is considered that the cation derived from the fluoride ion scavenger captures fluoride ions liberated from the fluoroisobutene derivative represented by the formula (3) during the reaction and precipitates the fluoride ions as a salt having low solubility in an organic solvent to promote the reaction, enabling the fluorine-containing pyrazolone compound represented by the formula (1a) to be obtained in a high yield.

In the presence of a nucleophilic reagent, 1-methylimidazole, the reaction in (c) above between the fluoroisobutene derivative represented by the formula (3) and the compound represented by the formula (6), is represented by the following reaction formula (C).

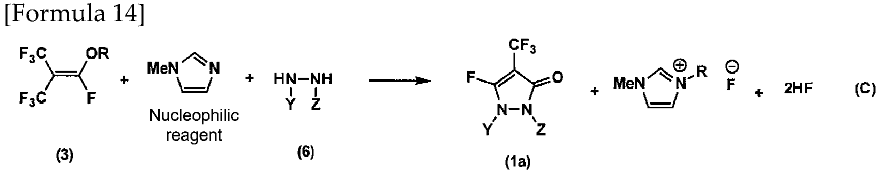

In the method for producing a fluorine-containing pyrazolone compound of another embodiment, the reaction (C) can be carried out in a single step. Consequently, the fluorine-containing pyrazolone compound of the formula (1a) can be obtained in a simplified manner. In the reaction of (c) above, a cyclic pyrazolone ring structure is formed between the fluoroisobutene derivative of the formula (3) and two nitrogen atoms derived from the compound of the formula (6), and -CF₃ and -F derived from the fluoroisobutene derivative are located at the 4- and 5-positions of the pyrazolone ring structure, respectively.

A reaction temperature during the reaction in (c) above is preferably - 20°C or higher and lower than the boiling point temperature of an organic solvent, more preferably 0 to 50°C, and still more preferably 10 to 30°C. A reaction time during the reaction in (c) above is preferably 0.5 to 48 hours, more preferably 1 to 36 hours, and still more preferably 10 to 25 hours. In the reaction of (c) above, the same hydrogen halide scavenger and solvent as in (a) above can be used.

A method for producing a fluorine-containing pyrazolone compound of another embodiment includes:
(d) a step of reacting a fluoroisobutene derivative represented by the following formula (3) with a compound represented by the following formula (5) and a compound represented by the following formula (6) or a salt thereof to obtain a fluorine-containing pyrazolone compound represented by the following formula (1b): 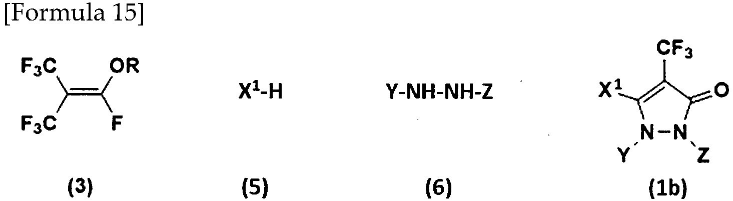
wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹, A², A³, and A⁴ each independently represent an organic group having 1 to 12 carbon atoms, and
R represents a hydrocarbon group having 1 to 12 carbon atoms.
A¹, A², A³, and A⁴ may be the same groups as A¹, A², A³, and A⁴ above.
Examples of R include a hydrocarbon group having 1 to 10 carbon atoms among A¹, A², and A³. More specifically, examples of R include a chain hydrocarbon group, aromatic hydrocarbon group, and alicyclic hydrocarbon group having 1 to 10 carbon atoms. Examples of the chain hydrocarbon group include alkyl groups such as a methyl group, an ethyl group, a n-propyl group, an i-propyl group, a n-butyl group, an i-butyl group, a sec-butyl group, a t-butyl group, a pentyl group, a hexyl group, a heptyl group, an octyl group, a nonyl group, and a decyl group; alkenyl groups such as an ethenyl group, a propenyl group, a butenyl group, a pentenyl group, a hexenyl group, a heptenyl group, an octenyl group, a nonenyl group, and a decenyl group; and alkynyl groups such as an ethynyl group, a propynyl group, a butynyl group, a pentynyl group, a hexynyl group, a heptynyl group, an octynyl group, a nonynyl group, and a decynyl group. Examples of the aromatic hydrocarbon group include a phenyl group. Examples of the alicyclic hydrocarbon group include a saturated or unsaturated cyclic hydrocarbon group, and examples of the cyclic hydrocarbon group include a cyclopropyl group, a cyclobutyl group, a cyclohexyl group, a cyclopentyl group, and an adamantyl group. Preferably, R is an alkyl group having 1 to 10 carbon atoms. When R is an alkyl group having 1 to 10 carbon atoms, a raw material of the fluorine-containing pyrazolone compound can be easily prepared.
Examples of salts of the compound represented by the formula (6) include hydrochloride, trifluoroacetate, and p-toluenesulfonate of the compound represented by the formula (6).

The step (d) of obtaining a fluorine-containing pyrazolone compound is preferably carried out in the presence of a nucleophilic reagent. Examples of the nucleophilic reagent include 1-methylimidazole, 4-dimethylaminopyridine, 1,4-diazabicyclo[2.2.2] octane, and triphenylphosphine. The use of the nucleophilic reagent can effectively eliminate the substituent -R from the fluoroisobutene derivative represented by the formula (3) to promote the formation of the fluorine-containing pyrazolone compound represented by the formula (1b). Preferably, the step (d) of obtaining a fluorine-containing pyrazolone compound is preferably carried out in the presence of a fluoride ion scavenger. It is preferable to react the fluoroisobutene derivative represented by the formula (3) with the compound represented by the formula (5) and the compound represented by the formula (6) or a salt thereof in the presence of a fluoride ion scavenger. The fluoride ion scavenger is not particularly limited as long as it is a substance having a function of capturing fluoride ions, and examples thereof include the same fluoride ion scavenger to be used in the step (a). It is considered that the cation derived from the fluoride ion scavenger captures fluoride ions liberated from the fluoroisobutene derivative represented by the formula (3) during the reaction and precipitates the fluoride ions as a salt having low solubility in an organic solvent to promote the reaction, enabling the fluorine-containing pyrazolone compound represented by the formula (1b) to be obtained in a high yield.

In the presence of a nucleophilic reagent, 1-methylimidazole, the reaction in (d) above between the fluoroisobutene derivative represented by the formula (3) and the compounds represented by the formulae (5) and (6), is represented by the following reaction formula (D).

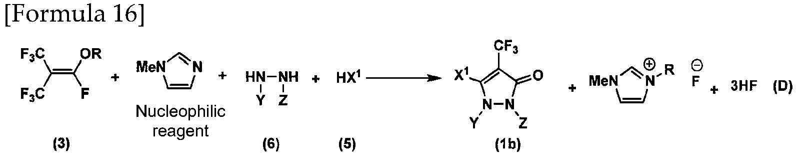

In the method for producing a fluorine-containing pyrazolone compound of another embodiment, the reaction (D) can be carried out in a single step. Consequently, the fluorine-containing pyrazolone compound of the formula (1b) can be obtained in a simplified manner. In the reaction of (d) above, a cyclic pyrazolone ring structure is formed between the fluoroisobutene derivative of the formula (3) and two nitrogen atoms derived from the compound of the formula (6), and -CF₃ derived from the fluoroisobutene derivative and -X¹ derived from the compound of the formula (5) are located at the 4- and 5-positions of the pyrazolone ring structure, respectively.

A reaction temperature during the reaction in (d) above is preferably - 20°C or higher and lower than the boiling point temperature of an organic solvent, more preferably 0 to 50°C, and still more preferably 10 to 30°C. A reaction time during the reaction in (d) above is preferably 0.5 to 48 hours, more preferably 1 to 36 hours, and still more preferably 10 to 25 hours. In the reaction of (d) above, the same hydrogen halide scavenger and solvent as in (a) above can be used.

A method for producing a fluorine-containing pyrazolone compound of another embodiment includes:
(e) a step of reacting a fluoroisobutane derivative represented by the following formula (4) with a compound represented by the following formula (6) or a salt thereof to obtain a fluorine-containing pyrazolone compound represented by the following formula (1a): 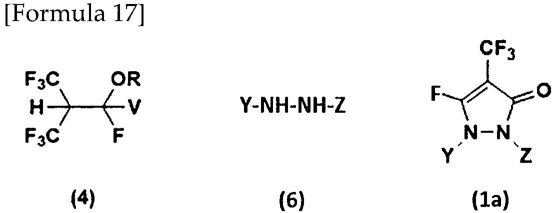
wherein
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
R represents a hydrocarbon group having 1 to 12 carbon atoms,
V represents a halogen atom, -OA⁵, or -SOₘA⁵, where m is an integer of 0 to 2, and
A¹ and A⁵ each independently represent an organic group having 1 to 12 carbon atoms.
A¹ and R may be the same groups as A¹ and R above.
V is preferably a halogen atom, and more preferably a fluorine atom. Examples of A⁵ include a methyl group, a phenyl group, and a trifluoromethyl group.
Examples of salts of the compound represented by the formula (6) include hydrochloride, trifluoroacetate, and p-toluenesulfonate of the compound represented by the formula (6).

The step (e) of obtaining a fluorine-containing pyrazolone compound is preferably carried out in the presence of a nucleophilic reagent. Examples of the nucleophilic reagent include 1-methylimidazole, 4-dimethylaminopyridine, 1,4-diazabicyclo[2.2.2]octane, and triphenylphosphine. The use of the nucleophilic reagent can effectively eliminate the substituent -R from the fluoroisobutane derivative represented by the formula (4) to promote the formation of the fluorine-containing pyrazolone compound represented by the formula (1a). Preferably, the step (e) of obtaining a fluorine-containing pyrazolone compound is preferably carried out in the presence of a fluoride ion scavenger. It is preferable to react the fluoroisobutane derivative represented by the formula (4) with the compound represented by the formula (6) or a salt thereof in the presence of a fluoride ion scavenger. The fluoride ion scavenger is not particularly limited as long as it is a substance having a function of capturing fluoride ions, and examples thereof include the same fluoride ion scavenger to be used in the step (a). It is considered that the cation derived from the fluoride ion scavenger captures fluoride ions liberated from the fluoroisobutane derivative represented by the formula (4) during the reaction and precipitates the fluoride ions as a salt having low solubility in an organic solvent to promote the reaction, enabling the fluorine-containing pyrazolone compound represented by the formula (1a) to be obtained in a high yield.

The reaction in (e) above between a fluoroisobutane derivative represented by the formula (4) and the compound represented by the formula (6) is represented by the following reaction formula (E).

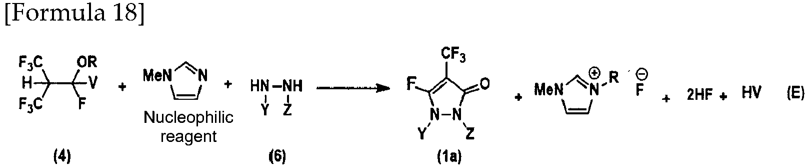

In the method for producing a fluorine-containing pyrazolone compound of another embodiment, the reaction (E) can be carried out in a single step. Consequently, the fluorine-containing pyrazolone compound of the formula (1a) can be obtained in a simplified manner. In the reaction of (e) above, a cyclic pyrazolone ring structure is formed between the fluoroisobutane derivative of the formula (4) and two nitrogen atoms derived from the compound of the formula (6), and -CF₃ and -F derived from the fluoroisobutane derivative are located at the 4- and 5-positions of the pyrazolone ring structure, respectively.

A reaction temperature during the reaction in (e) above is preferably - 20°C or higher and lower than the boiling point temperature of an organic solvent, more preferably 0 to 50°C, and still more preferably 10 to 30°C. A reaction time during the reaction in (e) above is preferably 0.5 to 48 hours, more preferably 1 to 36 hours, and still more preferably 10 to 25 hours. In the reaction of (e) above, the same hydrogen halide scavenger and solvent as in (a) above can be used.

A method for producing a fluorine-containing pyrazolone compound of another embodiment includes:
(f) a step of reacting a fluoroisobutane derivative represented by the following formula (4) with a compound represented by the following formula (5) and a compound represented by the following formula (6) or a salt thereof to obtain a fluorine-containing pyrazolone compound represented by the following formula (1b): 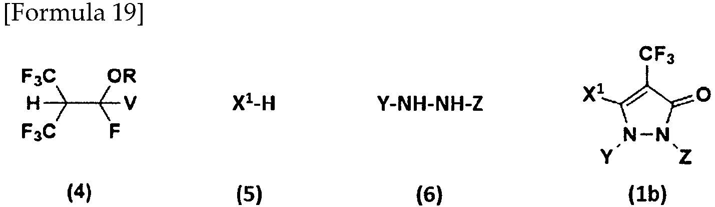
wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
R represents a hydrocarbon group having 1 to 12 carbon atoms,
V represents a halogen atom, -OA⁵, or -SOₘA⁵, where m is an integer of 0 to 2, and
A¹, A², A³, A⁴, and A⁵ each independently represent an organic group having 1 to 12 carbon atoms.
A¹, A², A³, A⁴, and R may be the same groups as A¹, A², A³, A⁴, and R above.
V is preferably a halogen atom, and more preferably a fluorine atom. Examples of A⁵ include a methyl group, a phenyl group, and a trifluoromethyl group.
Examples of salts of the compound represented by the formula (6) include hydrochloride, trifluoroacetate, and p-toluenesulfonate of the compound represented by the formula (6).

The step (f) of obtaining a fluorine-containing pyrazolone compound is preferably carried out in the presence of a nucleophilic reagent. Examples of the nucleophilic reagent include 1-methylimidazole, 4-dimethylaminopyridine, 1,4-diazabicyclo[2.2.2] octane, and triphenylphosphine. The use of the nucleophilic reagent can effectively eliminate the substituent -R from the fluoroisobutane derivative represented by the formula (4) to promote the formation of the fluorine-containing pyrazolone compound represented by the formula (1b). Preferably, the step (f) of obtaining a fluorine-containing pyrazolone compound is preferably carried out in the presence of a fluoride ion scavenger. It is preferable to react the fluoroisobutane derivative represented by the formula (4) with the compound represented by the formula (5) and the compound represented by the formula (6) or a salt thereof in the presence of a fluoride ion scavenger. The fluoride ion scavenger is not particularly limited as long as it is a substance having a function of capturing fluoride ions, and examples thereof include the same fluoride ion scavenger to be used in the step (a). It is considered that the cation derived from the fluoride ion scavenger captures fluoride ions liberated from the fluoroisobutane derivative represented by the formula (4) during the reaction and precipitates the fluoride ions as a salt having low solubility in an organic solvent to promote the reaction, enabling the fluorine-containing pyrazolone compound represented by the formula (1b) to be obtained in a high yield.

The reaction in (f) above between a fluoroisobutane derivative represented by the formula (4) and compounds represented by the formulae (5) and (6) is represented by the following reaction formula (F).

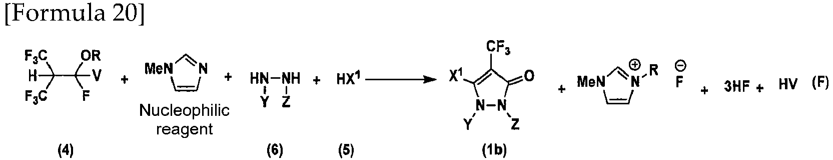

In the method for producing a fluorine-containing pyrazolone compound of another embodiment, the reaction (F) can be carried out in a single step. Consequently, the fluorine-containing pyrazolone compound of the formula (1b) can be obtained in a simplified manner. In the reaction of (f) above, a cyclic pyrazolone ring structure is formed between the fluoroisobutane derivative of the formula (4) and two nitrogen atoms derived from the compound of the formula (6), and -CF₃ derived from the fluoroisobutane derivative and -X¹ derived from the compound of the formula (5) are located at the 4- and 5-positions of the pyrazolone ring structure, respectively.

A reaction temperature during the reaction in (f) above is preferably - 20°C or higher and lower than the boiling point temperature of an organic solvent, more preferably 0 to 50°C, and still more preferably 10 to 30°C. A reaction time during the reaction in (f) above is preferably 0.5 to 48 hours, more preferably 1 to 36 hours, and still more preferably 10 to 25 hours. In the reaction of (f) above, the same hydrogen halide scavenger and solvent as in (a) above can be used.

Although embodiments of the present invention have been described above, the present invention is not limited to the above-described embodiments, and may be modified in various ways within the scope of the present invention, including all aspects included in the concepts and claims of the present invention.

### Examples

To further clarify the effects of the present invention, Examples will be described below, but the present invention is in no way limited to these Examples.

### (Example 1)

### Production of tert-butyl 5-fluoro-2-isopropyl-3-oxo-4-(trifluoromethyl)-1H-pyrazole-1-carboxylate

Under ice-water cooling, a solution 1 was prepared by adding 0.70 g (4.0 mmol) of tert-butyl 3-(isopropyl)carbazate to 30 g of THF. After a solution 2 was prepared by dissolving 1.3 g (4.0 mmol) of a salt composed of 3,3,3-trifluoro-2-(trifluoromethyl)propanoic acid fluoride anion and 1-methyl-4-dimethylaminopyridinium cation in 30 g of THF, the solution 2 was added dropwise to the solution 1 such that the internal temperature did not exceed 10°C, and the temperature was raised to room temperature. After about 16 hours, the resulting reaction mixture was added dropwise to a mixed solution of 1.0 g (8.0 mmol) of DIPEA and 30 g of THF under ice-water cooling such that the internal temperature did not exceed 10°C, and the temperature was raised to room temperature. After about 16 hours, the contents were subjected to silica gel column purification with a mixed solvent of hexane and ethyl acetate in a 7:3 ratio to produce tert-butyl 5-fluoro-2-isopropyl-3-oxo-4-(trifluoromethyl)-1H-pyrazole-1-carboxylate represented by the following formula (G). The yield of the resulting compound of the following formula (G) was 0.1 g, and the isolated yield thereof was 9%. In the present example, the salt composed of 3,3,3-trifluoro-2-(trifluoromethyl)propanoic acid fluoride anion and 1-methyl-4-dimethylaminopyridinium cation and tert-butyl 3-(isopropyl)carbazate correspond to the fluoroisobutanoic acid fluoride derivative of the formula (2) and the compound of the formula (6), respectively. 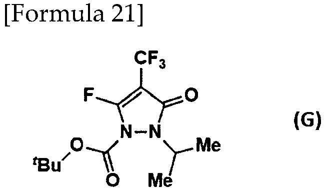 The analysis results of the resulting target product were as follows.
Mass Spectrum (APCI, m/z): 312 ([M]⁺)

### (Example 2)

### Production of N-((1-acetyl-5-oxo-2-phenyl-4-trifluoromethyl-1H-pyrazol-3-yl)oxy)-N-methylfuran-2-carboxamide

Under ice-water cooling, 10 g (43 mmol) of 1,1,1,3,3-pentafluoro-3-methoxy-2-trifluoromethyl-propane was dissolved in 80 g of THF to obtain a solution. Subsequently, 6 g (43 mmol) of diisopropylethylamine was added dropwise to the solution such that the internal temperature did not exceed 10°C, and the temperature was raised to room temperature. After about 1 hour, the resulting reaction mixture 1 was added dropwise to a mixed solution of 4.8 g (43 mmol) of quinuclidine and 20 g of THF under ice-water cooling such that the internal temperature did not exceed 10°C, and the temperature was raised to room temperature. After about 1 hour, the resulting reaction mixture 2 was added dropwise to a mixed solution of 5 g (43 mmol) of diethyleneglycol monomethyl ether and 20 g of THF under ice-water cooling such that the internal temperature did not exceed 10°C, and the temperature was raised to room temperature. After about 4 hours, the resulting reaction mixture 3 was added dropwise to a mixed solution of 6 g (43 mmol) of N-methylfurohydroxamic acid, 11 g (86 mmol) of diisopropylethylamine, and 20 g of THF under ice-water cooling such that the internal temperature did not exceed 10°C, and the temperature was raised to room temperature. After about 16 hours, the resulting reaction mixture 4 was added dropwise to a mixed solution of 7 g (43 mmol) of 1-acetyl-2-phenylhydrazine, 6 g (43 mmol) of diisopropylethylamine, and 20 g of THF under ice-water cooling such that the internal temperature did not exceed 10°C, and the temperature was raised to room temperature. After about 72 hours, to the resulting reaction mixture 5 were added 50 ml of diethyl ether and 50 ml of saturated saline, and the organic phase was dried over sodium sulfate. Thereafter, diethyl ether was distilled off under reduced pressure, and the residue was dissolved in ethyl acetate and subjected to silica gel column purification with a mixed solvent of hexane and ethyl acetate in a 7:3 ratio to produce N-((1-acetyl-5-oxo-2-phenyl-4-trifluoromethyl-1H-pyrazol-3-yl)oxy)-N-methylfuran-2-carboxamide represented by the following formula (H). The yield of the resulting compound of the following formula (H) was 0.5 g (isolated yield: 3%). In the present example, 1,1,1,3,3-pentafluoro-3-methoxy-2-trifluoromethyl-propane, N-methylfurohydroxamic acid, and l-acetyl-2-phenylhydrazine correspond to the fluoroisobutane derivative of the formula (4), the compound of the formula (5), and the compound of the formula (6), respectively. 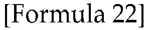 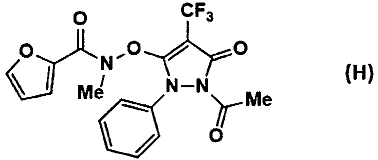 The analysis results of the resulting target product were as follows.
Mass Spectrum (APCI, m/z): 409 ([M]⁺)

### (Example 3)

### Production of 3-(bis(3-methoxypropyl)amino)-2-(trifluoromethyl)-5,6,7,8-tetrahydro-1H-pyrazolo[1,2-a]pyridazin-1-one

To a mixed solution of 3.0 g (30 mmol) of triethylamine and 20 g of THF under ice-water cooling was added 6.4 g (30 mmol) of 1,3,3,3-tetrafluoro-1-methoxy-2-trifluoromethyl-1-propene dropwise such that the internal temperature did not exceed 10°C, and the temperature was raised to room temperature. After about 1 hour, the resulting reaction mixture 1 was added dropwise to a mixed solution of 3.1 g (30 mmol) of 1-hexanol and 20 g of THF under ice-water cooling such that the internal temperature did not exceed 10°C, and the temperature was raised to room temperature. After about 4 hours, the resulting reaction mixture 2 was added dropwise to a mixed solution of 4.8 g (30 mmol) of bis(3-methoxypropyl)amine, 6.0 g (60 mmol) of triethylamine, and 20 g of THF under ice-water cooling such that the internal temperature did not exceed 10°C, and the temperature was raised to room temperature. After about 6 hours, the resulting reaction mixture 3 was added dropwise to a mixed solution of 4.8 g (30 mmol) of hexahydropyridazine dihydrochloride, 9.0 g (90 mmol) of diisopropylethylamine, and 20 g of methanol under ice-water cooling such that the internal temperature did not exceed 10°C, and the temperature was raised to room temperature. After about 36 hours, to the resulting reaction mixture 4 were added 50 ml of diethyl ether and 50 ml of saturated saline, and the organic phase was dried over sodium sulfate. Thereafter, diethyl ether was distilled off under reduced pressure, and the residue was dissolved in ethyl acetate and subjected to silica gel column purification with a mixed solvent of hexane and ethyl acetate in a 7:3 ratio to produce 3-(bis(3-methoxypropyl)amino)-2-(trifluoromethyl)-5,6,7,8-tetrahydro-1H-pyrazolo[1,2-a]pyridazin-1-one represented by the following formula (I). The yield of the resulting compound of the following formula (I) was 0.5 g (isolated yield: 5%). In this example, 1,3,3,3-tetrafluoro-1-methoxy-2-trifluoromethyl-1-propene, bis(3-methoxypropyl)amine, and hexahydropyridazine dihydrochloride correspond to the fluoroisobutene derivative of the formula (3), the compound of the formula (5), and the salt of the compound of the formula (6), respectively. 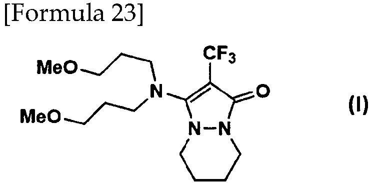 The analysis results of the resulting target product were as follows.
Mass Spectrum (APCI, m/z): 365 ([M]⁺)

### (Example 4)

### Production of 2-(1,7-dioxo-2-(trifluoromethyl)-6,7-dihydro-pyrazolo[1,2-a]pyrazol-3-yl)-1,1,3,3-tetramethylguanidine

To a mixed solution of 0.9 g (8.7 mmol) of triethylamine and 31 ml of THF under ice-water cooling was added 2.0 g (8.7 mmol) of 1,3,3,3-tetrafluoro-1-methoxy-2-trifluoromethyl-1-propene dropwise such that the internal temperature did not exceed 10°C, and the temperature was raised to room temperature. After about 1 hour, the resulting reaction mixture 1 was added dropwise to a mixed solution of 0.9 g (8.7 mmol) of 1-hexanol and 2 ml of THF under ice-water cooling such that the internal temperature did not exceed 10°C, and the temperature was raised to room temperature. After about 4 hours, the resulting reaction mixture 2 was added dropwise to 0.5 g (8.7 mmol) of 1,1,3,3-tetramethylguanidine and 0.9 g (8.7 mmol) of triethylamine under ice-water cooling such that the internal temperature did not exceed 10°C, and the temperature was raised to room temperature. After about 19 hours, the resulting reaction mixture 3 was cooled in ice water, 1.1 g (8.7 mmol) of 3-pyrazolidinone hydrochloride and 1.8 g (17.5 mmol) of triethylamine were added, and the temperature was raised to room temperature. After 71 hours, the resulting reaction mixture 4 was subjected to silica gel column purification to produce a trace amount of 2-(1,7-dioxo-2-(trifluoromethyl)-6,7-dihydro-pyrazolo[1,2-a]pyrazol-3-yl)-1,1,3,3-tetramethylguanidine represented by the following formula (J). In the present example, 1,3,3,3-tetrafluoro-1-methoxy-2-trifluoromethyl-1-propene, 1,1,3,3-tetramethylguanidine, and 3-pyrazolidinone hydrochloride correspond to the fluoroisobutene derivative of the formula (3), the compound of the formula (5), and the compound of the formula (6), respectively. 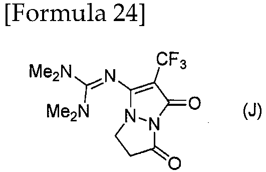 The analysis results of the resulting target product were as follows.
Mass Spectrum (APCI, m/z): 319.3 ([M]⁺)

### (Example 5)

### Production of tert-butyl 2-benzyl-5-(benzyl(propyl)amino)-3-oxo-4-(trifluoromethyl)-pyrazole-1-carboxylate

Under ice-water cooling, a solution 1 was prepared by adding 0.5 g (2.1 mmol) of tert-butyl-2-benzylhydrazine carboxylate to 20 g of THF. After a solution 2 was prepared by dissolving 0.6 g (2.1 mmol) of a salt composed of 3,3,3-trifluoro-2-(trifluoromethyl)propanoic acid fluoride anion and 1,3-dimethylimidazolium cation in 20 g of THF, the solution 2 was added dropwise to the solution 1 such that the internal temperature did not exceed 10°C, and the temperature was raised to room temperature. After about 16 hours, the resulting reaction mixture 1 was added dropwise to a mixed solution of 0.5 g (4.2 mmol) of diisopropylethylamine and 20 g of THF under ice-water cooling such that the internal temperature did not exceed 10°C, and the temperature was raised to room temperature. After about 47 hours, to the resulting reaction mixture 2 were added 0.3 g (2.1 mmol) of N-benzylpropan-1-amine and 0.3 g (2.1 mmol) of diisopropylethylamine dropwise at a room temperature, and the temperature was raised to 60°C. After about 16 hours, the resulting reaction mixture 3 was subjected to silica gel column purification to produce a trace amount of tert-butyl 2-benzyl-5-(benzyl(propyl)amino)-3-oxo-4-(trifluoromethyl)-pyrazole-1-carboxylate represented by the following formula (K). In the present example, the salt composed of 3,3,3-trifluoro-2-(trifluoromethyl)propanoic acid fluoride anion and 1,3-dimethylimidazolium cation, N-benzylpropan-1-amine, and tert-butyl-2-benzylhydrazine carboxylate correspond to the fluoroisobutanoic acid fluoride derivative of the formula (2), the compound of the formula (5), and the compound of the formula (6), respectively. 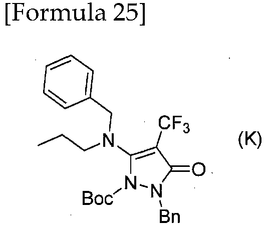 The analysis results of the resulting target product were as follows.
Mass Spectrum (APCI, m/z): 489.7 ([M]⁺)

### (Example 6)

### Production of 1,2-diethyl-5-(methyl(pyridin-2-ylmethyl)amino)-4-(trifluoromethyl)-pyrazol-3-one

To a mixed solution of 0.8 g (8.2 mmol) of triethylamine and 29 ml of THF under ice-water cooling was added 1.9 g (8.2 mmol) of 1,3,3,3-tetrafluoro-1-methoxy-2-trifluoromethyl-1-propene dropwise such that the internal temperature did not exceed 10°C, and the temperature was raised to room temperature. After about 1 hour, the resulting reaction mixture 1 was added dropwise to a mixed solution of 0.8 g (8.2 mmol) of 1-hexanol and 2 ml of THF under ice-water cooling such that the internal temperature did not exceed 10°C, and the temperature was raised to room temperature. After about 4 hours, the resulting reaction mixture 2 was added dropwise to 0.3 g (8.2 mmol) of 3-(methylaminomethyl)pyridine and 0.8 g (8.2 mmol) of triethylamine under ice-water cooling such that the internal temperature did not exceed 10°C, and the temperature was raised to room temperature. After about 17 hours, the resulting reaction mixture 3 was cooled in ice water, 1.3 g (8.2 mmol) of 1,2-diethylhydrazine dihydrochloride and 2.5 g (24.5 mmol) of triethylamine were added, and the temperature was raised to 50°C. After about 76 hours, the resulting reaction mixture 4 was subjected to silica gel column purification to produce a trace amount of 1,2-diethyl-5-(methyl(pyridin-2-ylmethyl)amino)-4-(trifluoromethyl)-pyrazol-3-one represented by the following formula (L). In the present example, 1,3,3,3-tetrafluoro-1-methoxy-2-trifluoromethyl-1-propene, 3-(methylaminomethyl)pyridine, and 1,2-diethylhydrazine dihydrochloride correspond to the fluoroisobutene derivative of the formula (3), the compound of the formula (5), and the compound of the formula (6), respectively. 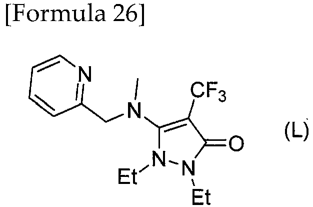 The analysis results of the resulting target product were as follows.
Mass Spectrum (APCI, m/z): 382.3 ([M]⁺)

### (Example 7)

### Production of 2-acetyl-5-(2-ethoxyethoxy)-1-phenyl-4-(trifluoromethyl)-pyrazol-3-one

Under ice-water cooling, 10 g (43 mmol) of 1,1,1,3,3-pentafluoro-3-methoxy-2-trifluoromethyl-propane was dissolved in 80 g of THF to obtain a solution. Subsequently, 6 g (43 mmol) of diisopropylethylamine was added dropwise to the solution such that the internal temperature did not exceed 10°C, and the temperature was raised to room temperature. After about 1 hour, the resulting reaction mixture 1 was added dropwise to a mixed solution of 4.8 g (43 mmol) of quinuclidine and 20 g of THF under ice-water cooling such that the internal temperature did not exceed 10°C, and the temperature was raised to room temperature. After about 1 hour, the resulting reaction mixture 2 was added dropwise to a mixed solution of 8 g (86 mmol) of 2-ethoxyethanol and 20 g of THF under ice-water cooling such that the internal temperature did not exceed 10°C, and the temperature was raised to room temperature. After about 30 minutes, to the resulting reaction mixture 3 was added 11 g (86 mmol) of diisopropylethylamine dropwise at a room temperature, and the temperature was raised to 60°C. After about 16 hours, the resulting reaction mixture 4 was added dropwise to a mixed solution of 7 g (43 mmol) of 1-acetyl-2-phenylhydrazine, 6 g (43 mmol) of diisopropylethylamine, and 20 g of THF at a room-temperature, and the temperature was raised to 60°C. After about 72 hours, to the resulting reaction mixture 5 were added 50 ml of diethyl ether and 50 ml of saturated saline, and the organic phase was dried over sodium sulfate. Thereafter, diethyl ether was distilled off under reduced pressure, and the residue was dissolved in ethyl acetate and subjected to silica gel column purification with a mixed solvent of hexane and ethyl acetate in a 7:3 ratio to produce 2-acetyl-5-(2-ethoxyethoxy)-1-phenyl-4-(trifluoromethyl)-pyrazol-3-one represented by the following formula (M). The yield of the resulting compound of the following formula (M) was 0.3 g (isolated yield: 2%). In the present example, 1,1,1,3,3-pentafluoro-3-methoxy-2-trifluoromethyl-propane, 2-ethoxyethanol, and 1-acetyl-2-phenylhydrazine correspond to the fluoroisobutane derivative of the formula (4), the compound of the formula (5), and the compound of the formula (6), respectively. 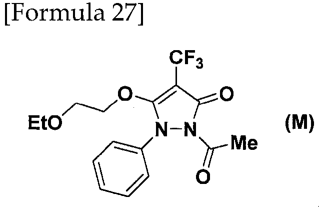 The analysis results of the resulting target product were as follows.
Mass Spectrum (APCI, m/z): 358.3 ([M]⁺)

## Claims

1. A fluorine-containing pyrazolone compound represented by the following formula (1): 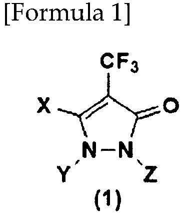 wherein
X represents a halogen atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring, and
A¹, A², A³, and A⁴ each independently represent an organic group having 1 to 12 carbon atoms.

2. A method for producing a fluorine-containing pyrazolone compound, comprising a step of reacting a fluoroisobutanoic acid fluoride derivative represented by the following formula (2) with a compound represented by the following formula (6) or a salt thereof to obtain a fluorine-containing pyrazolone compound represented by the following formula (1a): 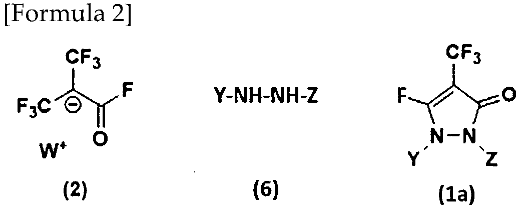 wherein
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹ represents an organic group having 1 to 12 carbon atoms, and
W⁺ represents an ammonium cation, an imidazolium cation, a pyridinium cation, or a phosphonium cation.

3. A method for producing a fluorine-containing pyrazolone compound, comprising a step of reacting a fluoroisobutanoic acid fluoride derivative represented by the following formula (2) with a compound represented by the following formula (5) and a compound represented by the following formula (6) or a salt thereof to obtain a fluorine-containing pyrazolone compound represented by the following formula (1b): 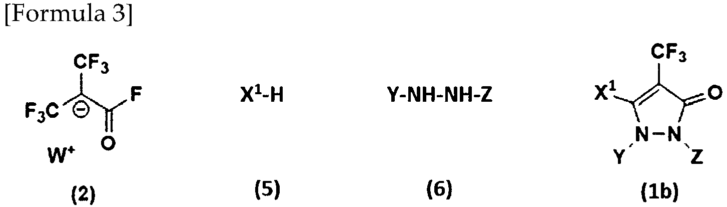 wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹, A², A³, and A⁴ each independently represent an organic group having 1 to 12 carbon atoms, and
W⁺ represents an ammonium cation, an imidazolium cation, a pyridinium cation, or a phosphonium cation.

4. A method for producing a fluorine-containing pyrazolone compound, comprising a step of reacting a fluoroisobutene derivative represented by the following formula (3) with a compound represented by the following formula (6) or a salt thereof to obtain a fluorine-containing pyrazolone compound represented by the following formula (1a): 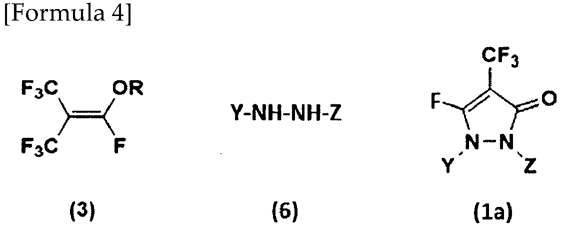 wherein
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹ represents an organic group having 1 to 12 carbon atoms, and
R represents a hydrocarbon group having 1 to 12 carbon atoms.

5. A method for producing a fluorine-containing pyrazolone compound, comprising a step of reacting a fluoroisobutene derivative represented by the following formula (3) with a compound represented by the following formula (5) and a compound represented by the following formula (6) or a salt thereof to obtain a fluorine-containing pyrazolone compound represented by the following formula (1b): 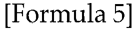 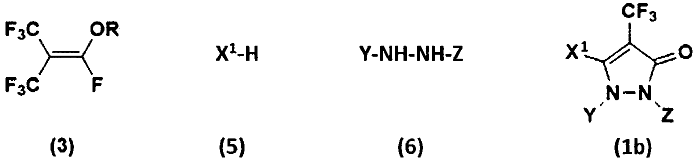 wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
A¹, A², A³, and A⁴ each independently represent an organic group having 1 to 12 carbon atoms, and
R represents a hydrocarbon group having 1 to 12 carbon atoms.

6. A method for producing a fluorine-containing pyrazolone compound, comprising a step of reacting a fluoroisobutane derivative represented by the following formula (4) with a compound represented by the following formula (6) or a salt thereof to obtain a fluorine-containing pyrazolone compound represented by the following formula (1a): 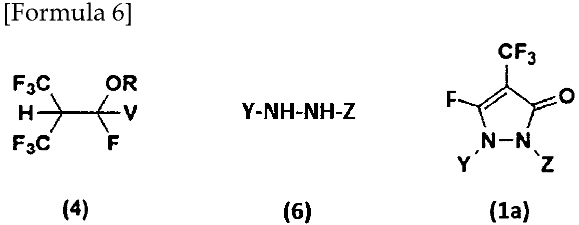 wherein
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
R represents a hydrocarbon group having 1 to 12 carbon atoms,
V represents a halogen atom, -OA⁵, or -SOₘA⁵, where m is an integer of 0 to 2, and
A¹ and A⁵ each independently represent an organic group having 1 to 12 carbon atoms.

7. A method for producing a fluorine-containing pyrazolone compound, comprising a step of reacting a fluoroisobutane derivative represented by the following formula (4) with a compound represented by the following formula (5) and a compound represented by the following formula (6) or a salt thereof to obtain a fluorine-containing pyrazolone compound represented by the following formula (1b): 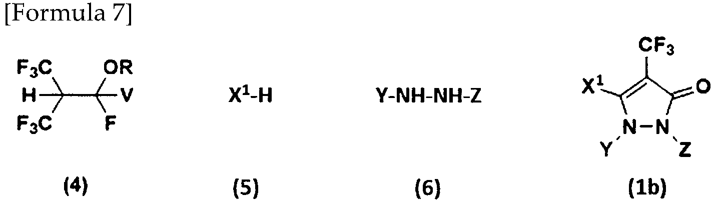 wherein
X¹ represents a halogen atom other than a fluorine atom, -OA¹, -S(O₁)A¹, where l is an integer of 0 to 2, -O-NA¹A², -NA¹A², -NA³-NA¹A², or -N=A⁴,
Y and Z each independently represent an aromatic hydrocarbon group having 1 to 12 carbon atoms, an alkyl group, -COA¹, -COOA¹, a benzyl group, or a carbamoyl group, or Y and Z are bonded to each other to form a ring,
R represents a hydrocarbon group having 1 to 12 carbon atoms,
V represents a halogen atom, -OA⁵, or -SOₘA⁵, where m is an integer of 0 to 2, and
A¹, A², A³, A⁴, and A⁵ each independently represent an organic group having 1 to 12 carbon atoms.
